# EUROPEAN PATENT SPECIFICATION

(11) **EP 3 957 282 B1**
(45) Date of publication and mention of the grant of the patent: **17.12.2025**
(21) Application number: 20791356.7
(22) Date of filing: 08.04.2020
(51) Int. Cl.: A61F 2/966, A61B 18/14, A61M 27/00, A61B 17/22, A61F 2/95, A61B 18/00

(54) **STENT DELIVERY DEVICE**
STENTEINFÜHRVORRICHTUNG
DISPOSITIF DE POSE DE STENT

(30) Priority: 18.04.2019 KR 20190045739
(43) Date of publication of application: 23.02.2022
(73) Proprietor: M.I.Tech Co., Ltd., Pyeongtaek-si, Gyeonggi-do 17706 (KR)
(72) Inventor: PARK, Hun Kuk, Pyeongtaek-si Gyeonggi-do 17873 (KR); KIM, Kyu Seok, Hwaseong-si Gyeonggi-do 18503 (KR); MOON, Jong Pil, Hwaseong-si Gyeonggi-do 18503 (KR); YUN, Ho, Asan-si Chungcheongnam-do 31412 (KR); KANG, Hye Don, Suwon-si Gyeonggi-do 16231 (KR); KIM, Beom Soon, Yongin-si Gyeonggi-do 17118 (KR); LEE, Young Eun, Yongin-si Gyeonggi-do 17006 (KR)
(74) Representative: dompatent
(86) International application number: PCT/KR2020/004739
(87) International publication number: WO 2020/213881

(56) References cited:
- EP-A1- 1 520 526
- EP-A1- 3 903 745
- WO-A1-2014/149022
- CN-A- 109 481 110
- GB-A- 2 474 252
- KR-A- 20130 095 317
- KR-A- 20160 003 460
- KR-A- 20170 095 733
- KR-B1- 101 886 551
- US-A1- 2017 035 426

## Description

### Technical Field

The present disclosure relates to a stent delivery device.

### Background Art

In general, a stent is a medical device that is inserted into a specific site inside the human body and expands and maintains a stenosed tube to its original size. In order to insert a stent into the human body, a method of placing a catheter loaded with a stent therein in the human body, discharging the stent out of the catheter and installing the stent at a specific site in the human body is widely used. In connection with this, Korean Utility Model Registration No. 20-0484392 has proposed a technology for a medical catheter capable of installing a stent.

Meanwhile, a pancreatic pseudocyst is an inflammatory cystic lesion that is caused by the leakage of pancreatic juice when the pancreatic duct is blocked and ruptured due to pancreatitis or pancreatic trauma or surgery. A pancreatic pseudocyst is a disease requiring prompt treatment because as the pseudocyst grows, it exerts pressure on the stomach or neighboring organs.

In order to treat such pseudocysts, there is used a procedure in which a pseudocyst is perforated and a stent is installed at a perforated site, thereby discharging the contents of the pseudocyst. However, a conventional catheter for the installation of a stent is not suitable for the perforation of a pseudocyst and the installation of a stent at a perforated site. Therefore, there is a need for the development of a technology that facilitates perforation and stent installation in the treatment of a pseudocyst.

### Disclosure

### Technical Problem

The technical spirit of the present disclosure is intended to overcome the above-described problems, and an object of the present disclosure is to provide a technology that facilitates the perforation of a pseudocyst and the installation of a stent at a perforated site during the treatment of the pseudocyst.

Objects to be achieved by the present invention are not limited to the above-described object, and other objects that are not mentioned will be clearly understood by those having ordinary skill in the art to which the present invention pertains from the following description.

### Technical Solution

In order to accomplish the above object, as an aspect of the present invention, there is provided a stent delivery device including: a housing configured such that a hollow is formed in the longitudinal direction thereof; a body part coupled to one side of the housing, and having a predetermined length; and a handle part coupled through the body part so that it can slide and move along the longitudinal direction of the body part; wherein a stopper configured to limit the sliding movement of the handle part is formed on a portion of the outer circumferential surface of the body part, and the limitation of movement applied by the stopper is released when the handle part is rotated by a predetermined angle along the circumferential direction of the outer circumferential surface, so that the sliding movement of the handle part is enabled.

Furthermore, the stent delivery device may further include a first tube coupled through the inside of the handle part, and a shaft configured such that a first tube passage is formed therein such that the first tube can move therethrough; a plurality of protrusion members may be formed on the outer circumferential surface of the shaft to be spaced apart from each other at predetermined intervals; and the housing may include a housing movement control member configured to control the forward/rearward movement of the housing by being selectively inserted into a separation space formed by the plurality of protrusion members.

In addition, the stent delivery device may further include a head part coupled to one side of the body part, and provided with an electrode therein, a second tube coupled through the first tube, and coupled to the head part at one end thereof, a third tube having a smaller diameter than the second tube, and coupled to the head part at one end thereof, and a tip part coupled to the other end of the third tube, and made of an insulating material; and a conductive member may be coupled to the outer circumferential surface of the tip part in order to transfer the current applied to the electrode.

Furthermore, the handle part may include a first tube fastening member coupled to at least a portion of the outer circumferential surface of the first tube while surrounding it; the first tube fastening member may have a fastening portion configured to fasten the first tube while surrounding it, and a leg portion formed to extend from the fastening portion; and a plurality of catch protrusions configured such that both ends of the leg portion can be caught thereon are formed on an inside of the handle part so that the first tube fastening member can also move when the handle part slides and moves.

In addition, a guide hole may be formed through the outer circumferential surface of the body part in the longitudinal direction of the body part, and the leg portion moves along the guide hole when the handle part slides and moves.

Moreover, the stopper may include a first stopper formed along the circumferential direction of the outer circumferential surface of the body part, and formed to protrude from a portion of the outer circumferential surface of the body part, and a second stopper spaced apart from the first stopper in the longitudinal direction of the body part, and formed to protrude from a portion of the outer circumferential surface of the body part; when viewed based on the circumference of the outer circumferential surface of the body part, the first stopper may protrude along a portion of the circumference of the outer circumferential surface of the body part, and the second stopper may protrude along another portion of the circumference of the outer circumferential surface of the body part; and a movement limitation member configured to be caught on the first stopper or the second stopper when the handle part slides and moves may be formed on the inside of the handle part.

The above-described technical solutions are merely exemplary, and should not be construed as limiting the present invention. In addition to the above-described exemplary embodiments, there may be one or more additional embodiments described in the drawings and the detailed description.

### Advantageous Effects

As described above, according to various embodiments of the present invention, the current applied to the electrode is transferred to the conductive member, so that it is easy for the tip part to perforate tissue. In other words, there is an effect in that the perforation of a stomach wall or a pancreas wall is performed easily.

Furthermore, when the stent is deployed at a perforated site, the limitation of movement applied by the first stopper is released through the rotation of the handle part, and thus the stent may be deployed primarily. When the handle part is rotated again after the stent has been deployed primarily, the limitation of movement applied by the second stopper is released, and thus the stent may be deployed secondarily.

More specifically, when a predetermined force is applied to the pressing depression formed on the outer circumferential surface of the handle part, the limitation of movement applied by the first stopper may be released as the handle part is rotated around the body part acting as an axis. Then, when the handle part is moved to a point between the first stopper and the second stopper, the first deployment of a stent is performed. Furthermore, when the handle part reaches the point between the first stopper and the second stopper, the limitation of movement applied by the second stopper is released by rotating the handle part again. Then, when the handle part is moved to the head part, the secondary deployment of the stent is performed. In this process, a user may easily release the limitation of movement applied by the stopper by rotating the handle part only with the force of the thumb, so that there is an advantage in that the operation thereof is convenient.

In addition, according to various embodiments of the present invention, the limitation of movement applied by each of the stoppers may be easily released through the rotation of the handle part 300 during the primary and secondary deployment of a stent. Accordingly, the installation of a stent is facilitated compared to the method of performing the secondary deployment of a stent by separating a safety device for preventing secondary deployment after the first deployment of the stent. Furthermore, a stent may be safely deployed without a separate safety device for preventing the deployment of a stent from occurring unexpectedly.

Effects according to various embodiments of the present invention are not limited to the effects mentioned above, and other effects that are not mentioned will be clearly understood by those of ordinary skill in the art from the description of the claims.

### Description of Drawings

FIG. 1 is a perspective view schematically showing a stent delivery device according to an embodiment of the present invention;
FIG. 2 is an exploded perspective view schematically showing the stent delivery device according to the embodiment of the present invention;
FIG. 3 is a partially sectional perspective view schematically showing the inside of a handle part according to the embodiment of the present invention;
FIG. 4 schematically shows a state in which the tip part of the stent delivery device according to the embodiment of the present invention is in contact with a stomach wall;
FIG. 5 schematically shows a state in which a stent is deployed primarily after a stent delivery device according to an embodiment of the present invention has perforated a stomach wall and a pseudocyst;
FIG. 6 schematically shows a state in which a stent primarily deployed by a stent delivery device according to an embodiment of the present invention is in close contact with the inner wall of a pseudocyst; and
FIG. 7 schematically shows a state in which the stent is deployed secondary by the stent delivery device according to the embodiment of the present invention.

### Mode for Invention

Preferred embodiments of the present invention will be described in detail below with reference to the accompanying drawings. For the sake of brevity of description, descriptions of well-known technical parts will be omitted or abridged.

It should be noted that references herein to "an" or "one" embodiment of the invention do not necessarily refer to the same embodiment but refer to at least one embodiment.

In the following embodiments, terms such as first, second, and the like are not used in limiting senses but are used for the purpose of distinguishing one component from another component.

In the following embodiments, a singular expression includes a plural expression unless the context clearly dictates otherwise.

In the following embodiments, terms such as include or have mean that one or more features or components described in the specification are present and do not preclude the possibility that one or more other features or components may be added.

In the following embodiments, when a part is described as being "connected" with another part, it includes not only the case where the former part is "directly connected" with the latter part, but also the case where the former part is "indirectly connected" with the latter part with another member interposed therebetween.

In the drawings, components may be exaggerated or reduced in size for convenience of description. For example, the size and thickness of each component shown in the drawings are arbitrarily shown for ease of description, and thus the present invention is not necessarily limited to what is shown.

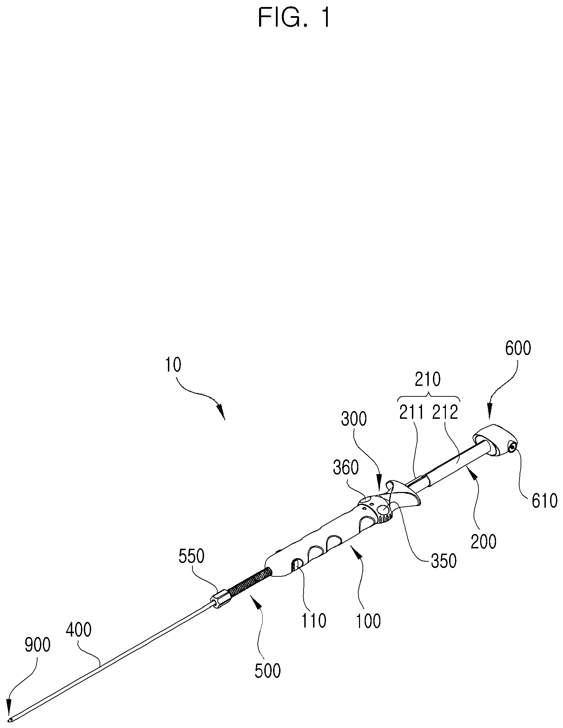

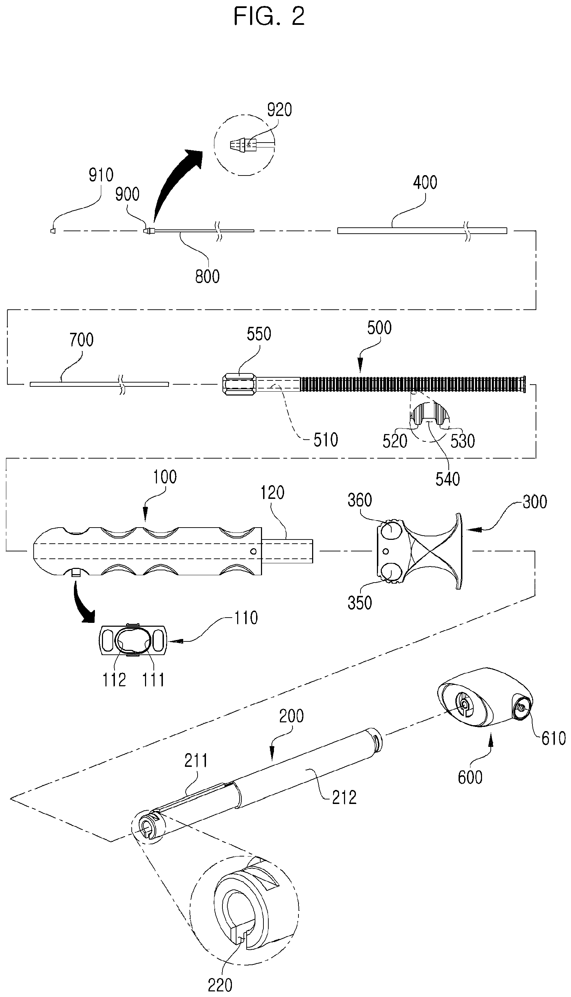

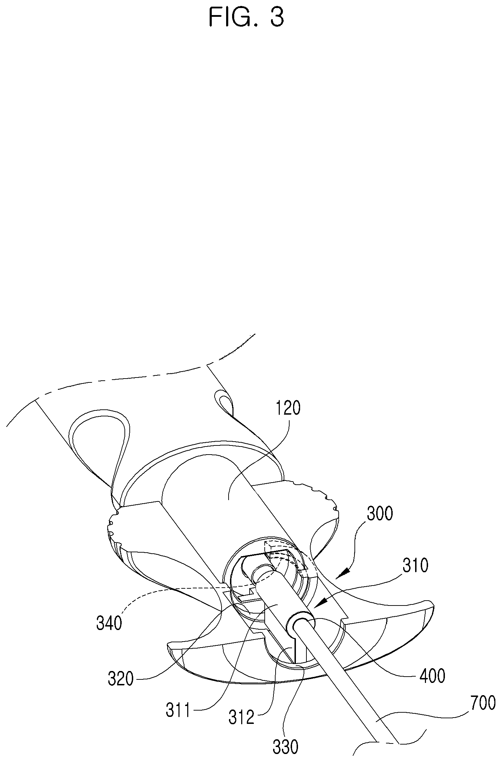

FIG. 1 is a perspective view schematically showing a stent delivery device according to an embodiment of the present invention, FIG. 2 is an exploded perspective view schematically showing the stent delivery device according to the embodiment of the present invention, and FIG. 3 is a partially sectional perspective view schematically showing the inside of a handle part according to the embodiment of the present invention.

Referring to FIGS. 1 to 3, the stent delivery device 10 according to the embodiment of the present invention may include a housing 100, a body part 200, a handle part 300, a first tube 400, a shaft 500, a head part 600, a second tube 700, a third tube 800, and a tip part 900.

First, the housing 100 is a body provided to be gripped by a user's hand, and a connection tube 120 may be extended from one side of the housing 100. A hollow is formed inside the housing 100 in the longitudinal direction of the housing 100. In an embodiment, a housing movement control member 110 may be inserted perpendicularly to the longitudinal direction of the housing 100. A first hole 111 having an inner diameter larger than the outer diameter of protrusion members 520 and 530 to be described later may be formed in the housing movement control member 110. Furthermore, a second hole 112 having an inner diameter smaller than the outer diameter of the protrusion members 520 and 530 may be formed in the housing movement control member 110 to be in contact with the first hole 111.

In an embodiment, the body part 200 may be implemented as a tube having a predetermined length. One side of the body part 200 may be coupled to the connection tube 120 of the housing 100, and the other side thereof may be coupled to the head part 600. In addition, a stopper 210 configured to limit the sliding movement of the handle part 300 to be described later may be formed on a portion of the outer circumferential surface of the body part 200.

The stopper 210 according to an embodiment may include a first stopper 211 and a second stopper 212. The first stopper 211 is formed along the circumferential direction of the outer circumferential surface of the body part 200, and may be formed to protrude in a portion of the outer circumferential surface of the body part 200. The second stopper 212 is formed to be spaced apart from the first stopper 211 in the longitudinal direction of the body part 200, and may be formed to protrude from a portion of the outer circumferential surface of the body part 200.

In this case, when viewed based on the circumference of the outer circumferential surface of the body 200, the first stopper 211 may protrude along a portion of the outer circumferential surface of the body 200, and the second stopper 212 may protrude along another portion of the outer circumferential surface of the body part 200.

Furthermore, a guide hole 220 may be formed long along the longitudinal direction of the body 200 on the lower side of the outer circumferential surface of the body part 200. In other words, the guide hole 220 may be formed in the range from one end of the body part 200 to the other end thereof.

The handle part 300 may slide along the longitudinal direction of the body part 200 in the state of being coupled through the body part 200. A space is formed in the handle part 300 into which at least a portion of the connection tube 120 and the body part 200 can be inserted. Accordingly, when the handle part 300 comes close to the housing 100, the connection tube 120 may be inserted into the inner space of the handle part 300.

In an embodiment, the first tube 400 may be coupled through the inside of the handle part 300. Furthermore, a first tube fastening member 310 may be disposed in the inner space of the handle part 300. The first tube fastening member 310 may surround at least a portion of the outer circumferential surface of the first tube 400 and maintain the state of being coupled to the first tube 400. More specifically, the first tube fastening member 310 may include a fastening portion 311 configured to surround and fasten the first tube 400, and a leg portion 312 configured to extend from the fastening portion 311 to one side.

In an embodiment, a plurality of catch protrusions 320 and 330 configured such that both ends of the leg portion 312 can be caught thereon are formed in the inner space of the handle part 300 so that when the handle part 300 slides and moves in the longitudinal direction of the body part 200, the first tube fastening member 310 moves together with the handle part 300. In other words, the leg portion 312 is located between the first catch protrusion 320 and the second catch protrusion 330. Accordingly, when the handle part 300 moves in the longitudinal direction of the body part 200, the leg part 312 may also move in the same direction as the handle part 300 moves.

In an embodiment, the guide hole 220 is formed through the outer circumferential surface of the body part 200 in the longitudinal direction of the body part 200 and the leg portion 312 maintains the state of being inserted into the guide hole 220, so that the leg portion 312 may move along the guide hole 220 when the handle part 300 slides and moves.

Furthermore, a movement limitation member 340 protruding along a portion of the inner circumferential surface may be installed in the inner space of the handle part 300. When the handle part 300 slides and moves in the longitudinal direction of the body part 200, the first or second stopper 211 or 212 and the movement limitation member 340 come into contact with and is caught on each other, so that the movement of the handle part 300 may be limited to a specific section.

However, when the handle part 300 is rotated by a predetermined angle along the circumferential direction of the outer circumferential surface, the limitation of movement applied by the first stopper 211 or the second stopper 212 is released, so that the sliding movement of the handle part 300 is enabled. For example, when the movement limitation member 340 is caught on the first stopper 211, the sliding movement of the handle part 300 is limited. In this case, when the handle part 300 is rotated by 180°, the limitation of movement applied by the first stopper 211 is released, so that the handle part 300 can slide and move to a point between the first stopper 211 and the second stopper 212. However, since the second stopper 212 and the movement limiting member 340 come into contact with and are caught on each other, the movement of the handle part 300 is limited again. In this case, when the handle part 300 is rotated by 180° again, the limitation of movement applied by the second stopper 212 is released, so that the handle part 300 can slide and move to the head part 600.

One or more pressing depressions 350 and 360 concavely depressed to be easily pressed with a thumb may be provided on the front surface of the outer circumferential surface of the handle part 300. Separate pressing depressions may also be formed on the rear surface of the handle unit 300 at locations corresponding to the pressing depressions 350 and 360 formed on the front surface of the handle unit 300.

In an embodiment, the shaft 500 is provided in a cylindrical shape, and the shaft 500 may be inserted into the hollow of the housing 100. A first tube passage 510 that is a space in which the first tube 400 can move is formed inside the shaft 500. Furthermore, a plurality of protrusion members 520 and 530 may be formed to be spaced apart from each other at predetermined intervals on the outer circumferential surface of the shaft 500. Each of the protrusion members 520 and 530 may be formed to protrude along the circumference of the outer circumferential surface of the shaft 500. The shaft 500 is coupled through the first hole 111 or second hole 112 of the housing movement control member 110 inserted into the housing 100.

Furthermore, the housing movement control member 110 may be selectively inserted into a separation space 540 formed by the plurality of protrusion members 520 and 530 to control the forward/rearward movement of the housing 100. For example, when the housing movement control member 110 is pressed in one direction, the first hole 111 comes close to the shaft 500. In contrast, when the housing movement control member 110 is pressed in the other direction, the second hole 112 comes close to the shaft 500. Even when the first hole 111 comes close to the shaft 500, the first hole 111 has an inner diameter larger than the outer diameter of the protrusion members 520 and 530, so that the housing 100 can move freely along the longitudinal direction of the shaft 500. In contrast, when the second hole 112 comes close to the shaft 500, the movement of the housing 100 is limited due to the insertion of the second hole 112 into the separation space 540, so that the housing 100 no longer moves.

A shaft fastening member 550 is a device configured to fasten the position of the shaft 500, and may be installed at one end of the shaft 500. A plurality of depressions and protrusions (not shown) that can be fastened to an endoscope channel inlet (not shown) may be formed on the inner circumferential surface of the shaft fastening member 550. In an embodiment, the shaft fastening member 550 may be integrated with the shaft 500. In one embodiment, the shaft fastening member 550 is coupled to the endoscope channel inlet through rotation, and the shaft 500 may also be rotated when the shaft fastening member 550 is rotated. Furthermore, the shaft 500 according to an embodiment has a cylindrical shape and the edge of the second hole 112 of the housing movement control member 110 is formed to be round, the second hole 112 is spaced apart from the space 540, so that the rotation of the shaft 500 can be performed freely even when the second hole 112 is inserted into the separation space 540 and the movement of the housing 100 is limited. Accordingly, regardless of the movement of the housing 500, the shaft 500 may be rotated and rotatably coupled to or separated from the endoscope channel inlet.

The head part 600 is coupled to the body part 200, and an electrode 610 may be provided inside the head part 600. The second tube 700 is coupled through the first tube 400, and one end of the second tube 700 may be coupled and fastened to the head part 600. The third tube 800 has a smaller diameter than the second tube 700, and is coupled through the second tube 700. One end of the third tube 800 may be coupled and fastened to the head part 600, and the tip part 900 may be coupled to the other end of the third tube 800.

In an embodiment, the tip part 900 has a truncated cone shape, and may be made of an insulating material (e.g., ceramic). Furthermore, a through hole 920 may be formed inside the tip part 900 to allow a guide wire 30 to be coupled therethrough.

In addition, a conductive member 910 may be coupled onto the outer circumferential surface of the tip part 900. The conductive member 910 according to an embodiment may be made of a stainless steel material. In an embodiment, an electric wire (not shown) having one end connected to the electrode 610 is connected to the conductive member 910 via the space between the second tube 700 and the third tube 800, so that the current applied to the electrode 610 may be transferred to the conductive member 910. In addition, in an embodiment, a copper wire may be applied as the electric wire.

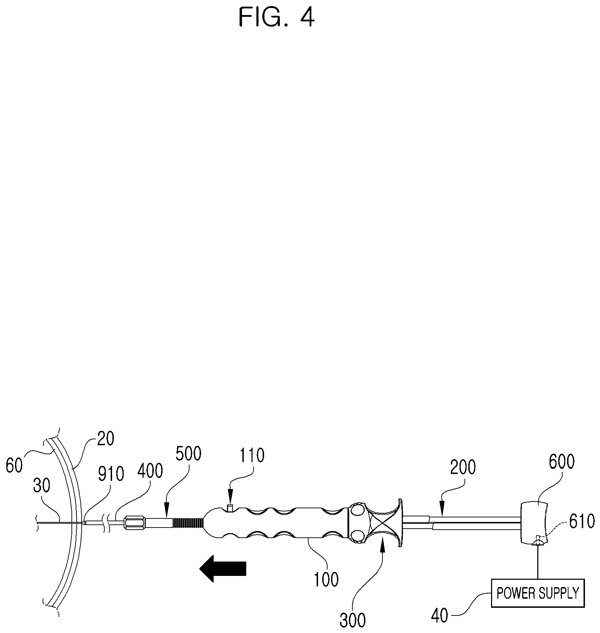

A process of installing the stent 50 using the stent delivery device 10 according to the embodiment of the present invention will be described as follows. First, FIG. 4 schematically shows a state in which the tip part 900 of the stent delivery device according to the embodiment of the present invention is in contact with a stomach wall 20. Referring to FIG. 4, the guide wire 30 passes through the inside of the first tube 400, and then passes through the through hole 920 formed inside the tip part 900. The tip part 900 may move along the guide wire 30 and approach the stomach wall 20. More specifically, when the housing movement control member 110 is pressed in one direction and then the position of the housing 100 is adjusted in the direction of the stomach wall 20, the conductive member 910 comes into contact with the stomach wall 20 as the tip part 900 moves toward the stomach wall 20. In this case, a power supply 40 is connected to the electrode 610. Accordingly, when current is supplied to the electrode 610 while the position of the housing 100 is further moved in the same direction, current is transferred to the conductive member 910 and the tip part 900 perforates the stomach wall 20 and a pseudocyst 60. After the tip part 900 has perforated the stomach wall 20 and the pseudocyst 60, the housing movement control member 110 is pressed in the other direction and thus the position of the housing 100 is fastened. Furthermore, the power supply 40 may be separated from the electrode 610.

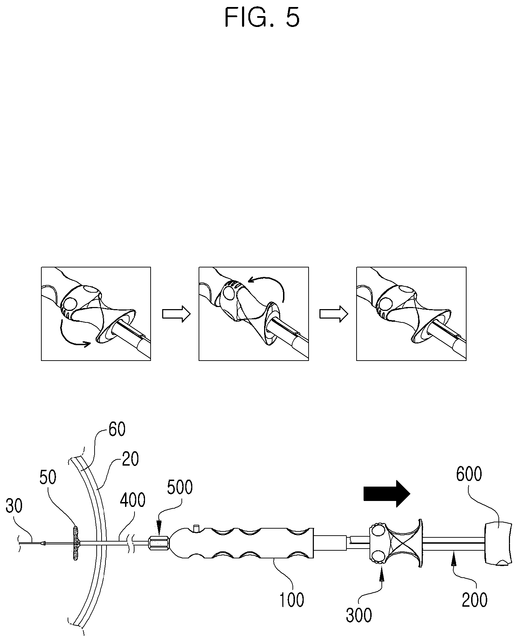

FIG. 5 schematically shows a state in which a stent is deployed primarily after a stent delivery device according to an embodiment of the present invention has perforated a stomach wall and a pseudocyst. Referring to FIG. 5, when the limitation of movement applied by the first stopper 211 is released by rotating the handle part 300 by 180° and then the handle part 300 is moved to a point between the first stopper 211 and the second stopper 212, the first tube 400 is also moved as the handle part 300 is moved. In this process, one end of the stent 50 loaded into the first tube 400 is exposed out of the first tube 400. In this case, the other end of the stent 50 maintains the state of being in close contact with the second tube 700, so that the stent 50 cannot be pushed back or moved together with the first tube 400 when the first tube 400 is moved, and thus the position of the stent 50 can be stably maintained.

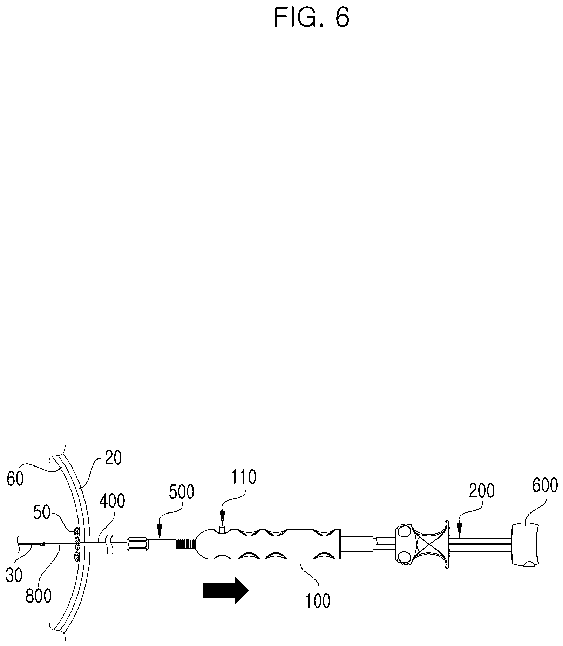

FIG. 6 schematically shows a state in which a stent primarily deployed by a stent delivery device according to an embodiment of the present invention is in close contact with the inner wall of a pseudocyst. Referring to FIG. 6, when the housing movement control member 110 is pressed in one direction and the position of the housing 100 is adjusted in the direction of the head 600, the stent 50 exposed out of the first tube 400 comes into close contact with the inner wall of the pseudocyst 60 while moving in the direction of the head part 600. When the stent 50 comes into close contact with the inner wall of the pseudocyst 60, the position of the housing 100 is fastened by pressing the housing movement control member 110 in the other direction.

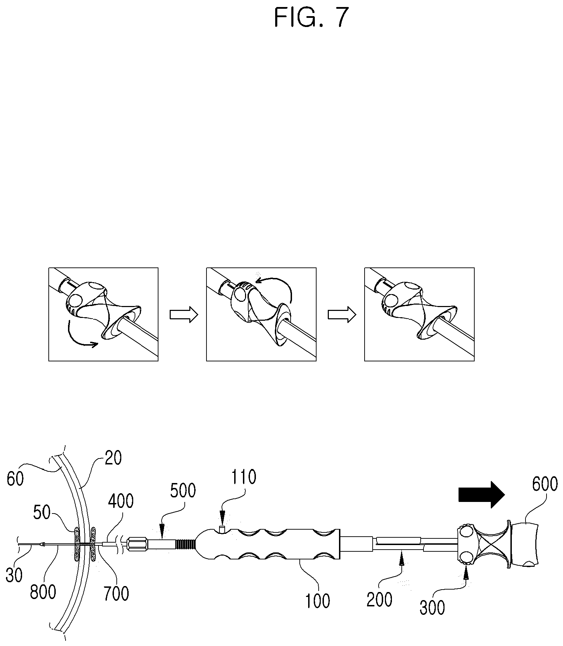

FIG. 7 schematically shows a state in which the stent is deployed secondary by the stent delivery device according to the embodiment of the present invention. Referring to FIG. 7, when the limitation of movement applied by the second stopper 212 has been released by rotating the handle part 300 located at a point between the first stopper 211 and the second stopper 212 by 180° and then the handle part 300 is moved in the direction of the head part 600, the first tube 400 is also moved as the handle part 300 is moved. In this process, the other end of the stent 50 loaded into the first tube 400 is completely exposed out of the first tube 400, and thus the installation of the stent 50 is completed.

As described above, according to various embodiments of the present invention, the current applied to the electrode 610 is transferred to the conductive member 910, so that it is easy for the tip part 900 to perforate tissue. In other words, there is an effect in that the perforation of the stomach wall 20 or a pancreas wall is performed easily.

Furthermore, when the stent 50 is deployed at a perforated site, the limitation of movement applied by the first stopper 211 is released through the rotation of the handle part 300, and thus the stent 50 may be deployed primarily. When the handle part 300 is rotated again after the stent 50 has been deployed primarily, the limitation of movement applied by the second stopper 212 is released, and thus the stent 50 may be deployed secondarily.

More specifically, when a predetermined force is applied to the pressing depression 350 or 360 formed on the outer circumferential surface of the handle part 300, the limitation of movement applied by the first stopper 211 may be released as the handle part 300 is rotated around the body part 200 acting as an axis. Then, when the handle part 300 is moved to a point between the first stopper 211 and the second stopper 212, the first deployment of the stent 50 is performed. Furthermore, when the handle part 300 reaches the point between the first stopper 211 and the second stopper 212, the limitation of movement applied by the second stopper 212 is released by rotating the handle part 300 again. Then, when the handle part 300 is moved to the head part 600, the secondary deployment of the stent 50 is performed.

The body part 200 is formed in a cylindrical shape so that the handle part 300 may be rotated easily, and thus a user may easily release the limitation of movement applied by the stopper 210 by rotating the handle part 300 only with the force of the thumb, so that there is an advantage in that the operation thereof is convenient.

In addition, according to various embodiments of the present invention, the limitation of movement applied by each of the stoppers 211 and 212 may be easily released through the rotation of the handle part 300 during the primary and secondary deployment of the stent 50. Accordingly, the installation of the stent 50 is facilitated compared to the method of performing the secondary deployment of the stent 50 by separating a safety device for preventing secondary deployment after the first deployment of the stent 50. Furthermore, the stent 50 may be safely deployed without a separate safety device for preventing the deployment of the stent 50 from occurring unexpectedly.

While the above-described detailed description of the present invention has been made based on the embodiments given with reference to the accompanying drawings, the above-described embodiments are merely preferred examples of the present invention. Therefore, it should not be understood that the present invention is limited only to the above-described embodiments, and the scope of the present invention should be understood as encompassing the attached claims and equivalents thereto.

### [Description of Reference symbols]

10: stent delivery device
100: housing
110: housing movement control member
111: first hole
112: second hole
120: connection tube
200: body part
210: stopper
211: first stopper
212: second stopper
220: guide hole
300: handle part
310: first tube fastening member
311: fastening portion
312: leg portion
320: first catch protrusion
330: second catch protrusion
340: movement limitation member
350, 360: pressing depression
400: first tube
500: shaft
510: first tube passage
520, 530: protrusion member
540: separation space
550: shaft fastening member
600: head part
610: electrode
700: second tube
800: third tube
900: tip part
910: conductive member
920: through hole
20: stomach wall
30: guide wire
40: power supply
50: stent
60: pseudocyst

## Claims

1. A stent delivery device (10) comprising:
a housing (100) configured such that a hollow is formed in a longitudinal direction thereof;
a body part (200) coupled to one side of the housing (100), and having a predetermined length; and
a handle part (300) coupled through the body part (200) so that it can slide and move along a longitudinal direction of the body part (200); and
a head part (600) coupled to one side of the body part (200), and provided with an electrode (610) therein
wherein a stopper (211, 212) configured to limit sliding movement of the handle part (300) is formed on a portion of an outer circumferential surface of the body part (200), and a limitation of movement applied by the stopper (211, 212) is released when the handle part (300) is rotated by a predetermined angle along a circumferential direction of the outer circumferential surface, so that sliding movement of the handle part (300) is enabled,
wherein
the stent delivery device (10) further comprises:
a first tube (400) coupled through an inside of the handle part (300); and
a shaft (500) configured such that a first tube passage (510) is formed therein such that the first tube (400) can move there through;
a plurality of protrusion members (520, 530) are formed on an outer circumferential surface of the shaft (500) to be spaced apart from each other at predetermined intervals; and
the housing (100) comprises a housing movement control member (110) configured to control forward/rearward movement of the housing (100) by being selectively inserted into a separation space formed by the plurality of protrusion members (520, 530),
wherein
the handle part (300) comprises a first tube fastening member (310) coupled to at least a portion of an outer circumferential surface of the first tube (400) while surrounding it;
the first tube fastening member (310) has a fastening portion (311) configured to fasten the first tube (400) while surrounding it, and a leg portion (312) formed to extend from the fastening portion (311); and
a plurality of catch protrusions (320, 330) configured such that both ends of the leg portion (312) can be caught thereon are formed on an inside of the handle part (300) so that the first tube fastening member can also move when the handle part (300) slides and moves,
wherein a guide hole (220) is formed through the outer circumferential surface of the body part (200) in the longitudinal direction of the body part (200), and the leg portion (312) moves along the guide hole when the handle part slides and moves,
wherein
the stopper (211, 212) comprises:
a first stopper (211) formed along the circumferential direction of the outer circumferential surface of the body part (200), and formed to protrude from a portion of the outer circumferential surface of the body part (200); and
a second stopper (212) spaced apart from the first stopper (211) in the longitudinal direction of the body part (200), and formed to protrude from a portion of the outer circumferential surface of the body part (200);
when viewed based on a circumference of the outer circumferential surface of the body part (200), the first stopper (211) protrudes along a portion of the circumference of the outer circumferential surface of the body part (200), and the second stopper (212) protrudes along another portion of the circumference of the outer circumferential surface of the body part (200); and
a movement limitation member (340) configured to be caught on the first stopper (211) or the second stopper (212) when the handle part (300) slides and moves is formed on an inside of the handle part (300), the limitation of movement applied by the first stopper (211) can be released as the handle part (300) is rotated around the body part (200) acting as an axis, and then when the handle part (300) is moved to a point between the first stopper (211) and the second stopper (212), the first deployment of a stent (50) is performed, and when the handle part (300) reaches the point between the first stopper (211) and the second stopper (212), the limitation of movement applied by the second stopper (212) can be released by rotating the handle part (300) again, and then then, when the handle part (300) is moved to the head part (600), the secondary deployment of the stent (50) is performed.

2. The stent delivery device of claim 1, wherein:
the stent delivery device (10) further comprises: ;
a second tube (700) coupled through the first tube (400), and coupled to the head part (600) at one end thereof;
a third tube (800) having a smaller diameter than the second tube (700), and coupled to the head part (600) at one end thereof; and
a tip part (900) coupled to a remaining end of the third tube (800), and made of an insulating material; and
a conductive member (910) is coupled to an outer circumferential surface of the tip part (900) in order to transfer a current applied to the electrode (610).

## Patentansprüche

1. Stent-Platzierungsvorrichtung (10), umfassend:
ein Gehäuse (100), das so konfiguriert ist, dass in seiner Längsrichtung ein Hohlraum entsteht;
ein Korpusteil (200), das mit einer Seite des Gehäuses (100) verbunden ist und eine vorgegebene Länge aufweist; und
ein Griffteil (300), das durch das Korpusteil (200) gekoppelt ist, so dass es entlang einer Längsrichtung des Korpusteils (200) gleiten und sich bewegen kann; und
ein Kopfteil (600), das mit einer Seite des Korpusteils (200) verbunden ist und eine darin befindliche Elektrode (610) aufweist,
wobei ein Anschlag (211, 212), der so konfiguriert ist, dass er die Gleitbewegung des Griffteils (300) begrenzen kann, an einem Abschnitt einer äußeren Umfangsfläche des Korpusteils (200) ausgebildet ist und eine durch den Anschlag (211, 212) ausgeübte Bewegungsbegrenzung aufgehoben wird, wenn das Griffteil (300) um einen vorbestimmten Winkel entlang einer Umfangsrichtung der äußeren Umfangsfläche gedreht wird, so dass die Gleitbewegung des Griffteils (300) ermöglicht wird,
wobei
die Stent-Platzierungsvorrichtung (10) weiterhin umfasst:
ein erstes Rohr (400), das über eine Innenseite des Griffteils (300) gekoppelt ist; und
eine Welle (500), die so konfiguriert ist, dass darin ein erster Rohrdurchgang (510) ausgebildet ist, durch den sich das erste Rohr (400) hindurch bewegen kann;
eine Vielzahl von Vorsprungselementen (520, 530) auf einer äußeren Umfangsfläche der Welle (500) so ausgebildet sind, dass sie in vorbestimmten Abständen voneinander beabstandet sind; und
das Gehäuse (100) ein Gehäusebewegungssteuerelement (110) umfasst, das so konfiguriert ist, dass es die Vorwärts-/Rückwärtsbewegung des Gehäuses (100) steuern kann, indem es selektiv in einen Trennraum eingesetzt wird, der durch die Vielzahl von Vorsprungselementen (520, 530) gebildet wird,
wobei
das Griffteil (300) ein erstes Rohrbefestigungselement (310) umfasst, das mit wenigstens einem Abschnitt einer Außenumfangsfläche des ersten Rohrs (400) verbunden ist und dieses umgibt;
das erste Rohrbefestigungselement (310) einen Befestigungsabschnitt (311) aufweist, der so konfiguriert ist, dass er das erste Rohr (400) befestigen kann, während er es umgibt, und einen Schenkelabschnitt (312), der so geformt ist, dass er sich vom Befestigungsabschnitt (311) weg erstreckt; und
an einer Innenseite des Griffteils (300) mehrere Rastvorsprünge (320, 330) ausgebildet sind, die so gestaltet sind, dass beide Enden des Schenkelabschnitts (312) daran rasten können, so dass sich das erste Rohrbefestigungselement auch bewegen kann, wenn das Griffteil (300) gleitet und sich bewegt,
wobei ein Führungsloch (220) durch die äußere Umfangsfläche des Korpusteils (200) in der Längsrichtung des Korpusteils (200) ausgebildet ist und sich der Schenkelabschnitt (312) entlang des Führungslochs bewegt, wenn das Griffteil gleitet und sich bewegt,
wobei
der Anschlag (211, 212) Folgendes umfasst:
einen ersten Anschlag (211), der entlang der Umfangsrichtung der Außenumfangsfläche des Korpusteils (200) ausgebildet ist und so ausgebildet ist, dass er von einem Abschnitt der Außenumfangsfläche des Korpusteils (200) wegragt; und
einen zweiten Anschlag (212), der in der Längsrichtung des Korpusteils (200) von dem ersten Anschlag (211) beabstandet ist ausgebildet ist und so ausgebildet ist, dass er von einem Abschnitt der Außenumfangsfläche des Korpusteils (200) wegragt;
bei Betrachtung basierend auf einem Umfang der äußeren Umfangsfläche des Korpusteils (200) der erste Anschlag (211) entlang eines Abschnitts des Umfangs der äußeren Umfangsfläche des Korpusteils (200) wegragt und der zweite Anschlag (212) entlang eines anderen Abschnitts des Umfangs der äußeren Umfangsfläche des Korpusteils (200) wegragt; und
ein Bewegungsbegrenzungselement (340), das so konfiguriert ist, dass es beim Gleiten und Bewegen des Griffteils (300) am ersten Anschlag (211) oder am zweiten Anschlag (212) hängen bleibt, an einer Innenseite des Griffteils (300) ausgebildet ist, die durch den ersten Anschlag (211) ausgeübte Bewegungsbegrenzung aufgehoben werden kann, wenn das Griffteil (300) um den als Achse fungierenden Korpusteil (200) gedreht wird und dann, wenn das Griffteil (300) zu einem Punkt zwischen dem ersten Anschlag (211) und dem zweiten Anschlag (212) bewegt wird, die erste Entfaltung eines Stents (50) durchgeführt wird, und wenn das Griffteil (300) den Punkt zwischen dem ersten Anschlag (211) und dem zweiten Anschlag (212) erreicht, die durch den zweiten Anschlag (212) ausgeübte Bewegungsbegrenzung durch erneutes Drehen des Griffteils (300) aufgehoben werden kann, und wenn das Griffteil (300) dann zum Kopfteil bewegt wird (600), die sekundäre Entfaltung des Stents (50) durchgeführt wird.

2. Stent-Platzierungsvorrichtung gemäß Anspruch 1, wobei:
die Stent-Platzierungsvorrichtung (10) weiterhin umfasst:
ein zweites Rohr (700), das über das erste Rohr (400) hindurch an einem Ende mit dem Kopfteil (600) verbunden ist;
ein drittes Rohr (800), das einen kleineren Durchmesser als das zweite Rohr (700) aufweist und an einem Ende mit dem Kopfteil (600) verbunden ist; und
ein Spitzenteil (900), das mit einem verbleibenden Ende des dritten Rohrs (800) verbunden ist und aus einem isolierenden Material besteht; und
ein leitfähiges Element (910) mit einer äußeren Umfangsfläche des Spitzenteils (900) gekoppelt ist, um einen an die Elektrode (610) angelegten Strom zu übertragen.

## Revendications

1. Dispositif de pose de stent (10), comprenant :
un boîtier (100) configuré de telle sorte qu'un creux est formé dans une direction longitudinale de celui-ci ;
une partie de corps (200) couplée à un côté du boîtier (100), et ayant une longueur prédéterminée ; et
une partie de poignée (300) couplée à travers la partie de corps (200) de sorte qu'elle puisse glisser et se déplacer le long d'une direction longitudinale de la partie de corps (200) ; et
une partie de tête (600) couplée à un côté de la partie de corps (200) et pourvue d'une électrode (610) à l'intérieur,
dans lequel une butée (211, 212) configurée pour limiter le mouvement de coulissement de la partie de poignée (300) est formée sur une partie d'une surface circonférentielle extérieure de la partie de corps (200), et une limitation de mouvement appliquée par la butée (211, 212) est libérée lorsque la partie de poignée (300) est tournée d'un angle prédéterminé le long d'une direction circonférentielle de la surface circonférentielle extérieure, de sorte que le mouvement de coulissement de la partie de poignée (300) est activé,
dans lequel
le dispositif de pose de stent (10) comprend en outre :
un premier tube (400) couplé à travers l'intérieur de la partie de poignée (300) ; et
un arbre (500) configuré de telle sorte qu'un premier passage de tube (510) y est formé de telle sorte que le premier tube (400) puisse s'y déplacer ;
une pluralité d'éléments en saillie (520, 530) sont formés sur une surface circonférentielle extérieure de l'arbre (500) pour être espacés les uns des autres à des intervalles prédéterminés ; et
le boîtier (100) comprend un élément de commande de mouvement du boîtier (110) configuré pour commander le mouvement vers l'avant/vers l'arrière du boîtier (100) en étant inséré sélectivement dans un espace de séparation formé par la pluralité d'éléments en saillie (520, 530),
dans lequel
la partie de poignée (300) comprend un premier élément de fixation de tube (310) couplé à au moins une partie d'une surface circonférentielle extérieure du premier tube (400) tout en l'entourant ;
le premier élément de fixation de tube (310) comporte une partie de fixation (311) configurée pour fixer le premier tube (400) tout en l'entourant, et une partie de jambe (312) formée pour s'étendre à partir de la partie de fixation (311) ; et
une pluralité de saillies d'accrochage (320, 330) configurées de telle sorte que les deux extrémités de la partie de jambe (312) peuvent être accrochées sur celles-ci sont formées à l'intérieur de la partie de poignée (300) de sorte que le premier élément de fixation de tube peut également se déplacer lorsque la partie de poignée (300) coulisse et se déplace,
dans lequel un trou de guidage (220) est formé à travers la surface circonférentielle extérieure de la partie de corps (200) dans la direction longitudinale de la partie de corps (200), et la partie de jambe (312) se déplace le long du trou de guidage lorsque la partie de poignée coulisse et se déplace,
dans lequel
la butée (211, 212) comprend :
une première butée (211) formée le long de la direction circonférentielle de la surface circonférentielle extérieure de la partie de corps (200), et formée pour faire saillie à partir d'une partie de la surface circonférentielle extérieure de la partie de corps (200) ; et
une deuxième butée (212) espacée de la première butée (211) dans la direction longitudinale de la partie de corps (200), et formée pour faire saillie à partir d'une partie de la surface circonférentielle extérieure de la partie de corps (200) ;
lorsqu'il est observé sur la base d'une circonférence de la surface circonférentielle extérieure de la partie de corps (200), la première butée (211) fait saillie le long d'une partie de la circonférence de la surface circonférentielle extérieure de la partie de corps (200), et la deuxième butée (212) fait saillie le long d'une autre partie de la circonférence de la surface circonférentielle extérieure de la partie de corps (200) ; et
un élément de limitation de mouvement (340) configuré pour être accroché à la première butée (211) ou à la deuxième butée (212) lorsque la partie de poignée (300) coulisse et se déplace est formé à l'intérieur de la partie de poignée (300), la limitation de mouvement appliquée par la première butée (211) peut être relâchée lorsque la partie de poignée (300) est tournée autour de la partie de corps (200) agissant comme un axe, et ensuite, lorsque la partie de poignée (300) est déplacée vers un point situé entre la première butée (211) et la deuxième butée (212), le premier déploiement d'un stent (50) est effectué, et lorsque la partie de poignée (300) atteint le point situé entre la première butée (211) et la deuxième butée (212), la limitation de mouvement appliquée par la seconde butée (212) peut être relâchée en faisant à nouveau tourner la partie de poignée (300), et ensuite, lorsque la partie de poignée (300) est déplacée vers la partie de tête (600), le déploiement secondaire du stent (50) est effectué.

2. Dispositif de pose de stent selon la revendication 1, dans lequel :
le dispositif de pose de stent (10) comprend en outre :
un deuxième tube (700) couplé à travers le premier tube (400), et couplé à la partie de tête (600) à une extrémité de celui-ci ;
un deuxième tube (800) couplé à travers le premier tube (700), et couplé à la partie de tête (600) à une extrémité de celui-ci ;
une partie de pointe (900) couplée à une extrémité restante du troisième tube (800), et constituée d'un matériau isolant ; et
un élément conducteur (910) est couplé à une surface circonférentielle extérieure de la partie de pointe (900) afin de transférer un courant appliqué à l'électrode (610).
